**Europäisches Patentamt**

(19) **European Patent Office**

**Office européen des brevets**

(11) Veröffentlichungsnummer: **0 082 359**
**B1**

(12) **EUROPÄISCHE PATENTSCHRIFT**

(45) Veröffentlichungstag der Patentschrift:
19.02.86

(51) Int. Cl.⁴: **C 07 K 3/20,** C 07 G 15/00

(21) Anmeldenummer: 82111061.6

(22) Anmeldetag: 30.11.82

(54) **Verfahren zur Trennung von Gemischen aus Insulin, Insulinderivaten und gegebenenfalls Verunreinigungen.**

(30) Priorität: 03.12.81 DE 3147842

(43) Veröffentlichungstag der Anmeldung:
29.06.83 Patentblatt 83/26

(45) Bekanntmachung des Hinweises auf die Patenterteilung:
19.02.86 Patentblatt 86/8

(84) Benannte Vertragsstaaten:
AT BE CH DE FR GB IT LI NL SE

(56) Entgegenhaltungen:
Chemical Abstracts Band 84, Nr.23, 7. Juni 1976
Columbus, Ohio, USA L.R.Pickart et al. "Purification of
growth-promoting peptides and proteins, and of
histones, by high pressure silica gel chromatography"
Seite 181, Spalten 1,2, Abstract Nr.161310a.
(73) Patentinhaber: **HOECHST AKTIENGESELLSCHAFT,
Postfach 80 03 20, D-6230 Frankfurt am Main 80 (DE)**

(72) Erfinder: **Obermeier, Rainer, Dr., Langenhainer Weg 14,
D-6234 Hattersheim am Main (DE)**
Erfinder: **Teetz, Volker, Dr., An der Tann 20,
D-6238 Hofheim am Taunus (DE)**
Erfinder: **Ludwig, Jürgen, Ringstrasse 13,
D-6486 Brachttal (DE)**

## Beschreibung

Es ist bekannt, natürliches Schweineinsulin durch chemischen oder enzymatischen Austausch des Alanins in Position B 30 gegen Threonin auf semisynthetischem Wege in Humaninsulin umzuwandeln.

Obermeier, R., Geiger, R. Hoppe-Seyler's Z.physiol. Chemie 357, 759–767 (1976).

Ruttenberg, M. A. Science 177 623–626 (1972).

Morihara, K., Oka, T., Tsuzuki, H. Nature 280 412–413 (1979).

Inouye, K. J. Am. Chem. Soc. 101 751–752 (1979).

Schmitt, E., Gattner, H.G. Hoppe-Seyler's Z.physol. Chem. 359 799–802 (1978).

Bromer, W.W., Chance, R.E. Biochem. Biophys. Acta 133 219–223 (1967).

Ziel dieser Umwandlung ist, bei der Behandlung des menschlichen Diabetes mellitus aus immunologischen Gründen speziesgleiches Insulin einsetzen zu können.

Die Vorteile dieser Behandlung können nur dann zur Geltung kommen, wenn es gelingt, aus dem Rohmaterial der Semisynthese Insulinderivate und Verunreinigungen sicher vom Humaninsulin abzutrennen.

Die bekannten Trennmethoden sind nicht geeignet, mit Sicherheit die Abtrennung des Humaninsulins von Schweineinsulinkomponenten zu gewährleisten. Aus den in der Literatur veröffentlichten Aminosäureanalysen ist abzuleiten, dass im semisynthetischen Humaninsulin Kontamination mit Schweineinsulin bis zu einer Grössenordnung von 25% möglich ist.

Um selbst spurenweise Verunreinigungen von Humaninsulin mit Schweineinsulin oder Enzymen durch die wiederholte Verwendung regenerierbarer Füllmaterialien für Chromatographiesäulen zu vermeiden, ist ein billiges Einwegmaterial mit optimaler Trennleistung als Säulenfüllung wünschenswert. In der analytischen Hochdruckflüssigkeitschromatographie werden hochauflösende chromatographische Trennungen ausschliesslich an hydrophobem, mit organischen Resten derivatisiertem Kieselgel («reversed phase») erzielt. Die hohen Kosten für dieses Material verbieten jedoch den Gebrauch als Einwegsäulenfüllmengen.

Es wurde nun gefunden, dass die chromatographische Trennung von Rohgemischen aus enzymatischen Semisynthesen von Insulin an Säulen, gepackt mit käuflichem Kieselgel bei Verwendung bestimmter organischer Lösungsmittelgemische wie beispielsweise Chloroform: Methanol: Wasser: Triethylamin: Ameisensäure allen anderen Trennmethoden überlegen ist. Diese Überlegenheit resultiert einerseits aus den zum Teil grossen Unterschieden in den Retentionszeiten der Komponenten der zu trennenden Gemische, andererseits in der Schnelligkeit eines Säulenlaufs. Die Kosten des nach einem Lauf verworfenen Kieselgels können im Vergleich zum verarbeiteten Insulin vernachlässigt werden.

Gegenstand der Erfindung ist somit ein Verfahren zur chromatographischen Trennung von Gemischen aus Insulin, Insulinderivaten und ggf. Verunreinigungen, das dadurch gekennzeichnet ist, dass man die Gemische an einer mit Kieselgel gefüllten Säule chromatographiert, wobei das Kieselgel nicht mit organischen Resten derivatisiert ist und eine Korngrösse ≥ 40 µm aufweist.

Gegenstand der Erfindung ist auch ein Insulin, insbesondere Human- oder Schweineinsulin bzw. ein Insulinester, wie insbesondere Humaninsulin-B30-di-tert-butyl-threonin.

Das Verfahren eignet sich insbesondere zur Reinigung von Insulin, und zur Trennung von Insulin, insbesondere Humaninsulin oder Schweineinsulin oder seinen Abbauprodukten von Trypsin oder ähnlichen Fermenten und Insulinestern oder anderen Insulin-Derivaten.

Als Säulenfüllung kommt jedes käufliche Kieselgel in Betracht, beispielsweise das Handelsprodukt $SiO_260$ von Merck & Co., Darmstadt, vorzugsweise ein solches der Korngrösse 63–125 µm. Vorteilhaft weist es einen mittleren Porendurchmesser von 60 Å auf.

Als Laufmittel können zur Chromatographie alle diejenigen Laufmittelgemische verwendet werden, in denen Insulinester oder -derivate einerseits und Insulin oder dessen Abbauprodukte andererseits auf käuflichen Dünnschichtchromatographie-Platten unterschiedliche Rf-Werte aufweisen.

Besonders gute Trenneffekte werden erreicht, wenn man das Rohprodukt in einem Laufmittelgemisch chromatographiert, das aus 1500–2100 Volumenteilen Chloroform, 1000–1500 Volumenteilen Methanol, 350–450 Volumenteilen Wasser, 35–55 Volumenteilen Triethylamin und 0–15 Volumenteilen Ameisensäure besteht.

Besonders vorteilhaft ist das Verfahren bei der Abtrennung des Humaninsulin-B 30-di-tert-butyl-threonin von Schweineinsulin und Trypsin, ein Gemisch, wie es bei der enzymatischen Umwandlung von Schweineinsulin in Humaninsulin vorkommt.

Anwendungsbeispiele

1.4 g eines Gemisches aus 70% Humaninsulin-B 30-di-tert-butyl-threonin und 30% Schweineinsulin werden in 20 ml eines Lösungsmittels bestehend aus Chloroform: Methanol: Wasser: Triethylamin: Ameisensäure = 1800: 1500: 375: 45: 9 (v/v) gelöst und mit Hilfe einer Einwegspritze auf eine trockene Lobar[(R)] Fertigsäule, Grösse C aufgegeben. Die Säule wird mit dem gleichen Lösungsmittel entwickelt. Nach dem Erscheinen der Lösungsmittelfront werden einzelne Fraktionen (10 ml) gesammelt.

Humaninsulin-B30-(But)$_2$ erscheint in den Fraktionen 37–53. Diese werden vereinigt, am Rotationsverdampfer auf ein Volumen von 30–50 ml eingeengt. Zu dieser Lösung werden etwa 500 ml Aceton gegeben und das präzipitierte Produkt durch Zentrifugation isoliert.

Das Schweineinsulin wird in den Fraktionen 120–137 eluiert und wie oben beschrieben isoliert. Beide Produkte werden in leichtem Vakuum getrocknet.

Ausbeute: 2,59 g Humaninsulin-B30-(But)$_2$
1,03 g Insulin (S)

2. N$\alpha$B$_1$-tert-butyloxycarbonyl-B30-di-tert-butyl-threonin-Insulin (4 g) werden wie ein Beispiel 1 gelöst und auf eine mit 300 g Kieselgel SiO$_2$–60$^{(R)}$ gefüllte Säule (2,5 × 59 cm), die mit Chloroform äquilibriert wurde, aufgegeben. Die Säule wird mit dem gleichen Lösungsmittelgemisch wie in Beispiel 1 eluiert. Der zuerst erscheinende Peak enthält B$_1$-BOC-Insulin-B30-(But)$_2$, Schweineinsulin wird in den späten Fraktionen eluiert. Die Aufarbeitung geschieht wie in Beispiel 1.

3. Ein Gemisch wie im Beispiel 1 (4 g) wird auf eine Lobar$^{(R)}$ Fertigsäule C äquilibriert mit 70% Ethanol/30% Tris-Puffer (0,05 M pH 8,0), aufgetragen und mit demselben Elutionsmittel chromatographiert. Die entsprechenden Fraktionen, die voneinander abgetrennt, Humaninsulin B30-di-tert-threonin und Schweineinsulin enthalten, werden wie in Beispiel 1 aufgearbeitet.

4. Ungereinigtes Schweineinsulin (4 g) wird in einem Gemisch Chloroform: Methanol: Wasser: Triethylamin: Ameisensäure = 1200: 1100: 370: 47: 11 gelöst und wie in Beispiel 2 über eine Kieselsäule chromatographiert. Das in 10 ml Portionen fraktionierte Eluat wird wie in Beispiel 1 aufgearbeitet. Fraktionen, die nach Überprüfung in HPLC und Polyacrylamid-Gelelektrophorese reines Schweineinsulin enthalten, werden vereinigt. Ausbeute: 3,61 g.

## Patentansprüche

1. Verfahren zur chromatographischen Trennung von Gemischen aus Insulin, Insulinderivaten und ggf. Verunreinigungen, dadurch gekennzeichnet, dass man die Gemische an einer mit Kieselgel gefüllten Säule chromatographiert, wobei das Kieselgel nicht mit organischen Resten derivatisiert ist und eine Korngrösse $\geq$ 40 $\mu$m aufweist.

2. Verfahren gemäss Anspruch 1, dadurch gekennzeichnet, dass das verwendete Kieselgel eine Korngrösse 63–125 $\mu$m aufweist.

3. Verfahren gemäss Anspruch 1 oder 2, dadurch gekennzeichnet, dass das verwendete Kieselgel einen mittleren Porendurchmesser von 60 Å aufweist.

4. Verfahren gemäss einem der Ansprüche 1 bis 3, dadurch gekennzeichnet, dass mit einem Laufmittelgemisch aus Chloroform, Methanol, Wasser, Triethylamin und ggf. Ameisensäure chromatographiert wird, wobei das Laufmittelgemisch vorteilhaft aus 1500–2100 Volumenteilen Chloroform, 1000–1500 Volumenteilen Methanol, 350–450 Volumenteilen Wasser, 35–55 Volumenteilen Triethylamin und 0–15 Volumenteilen Ameisensäure besteht.

5. Verfahren gemäss einem der Ansprüche 1 bis 4, dadurch gekennzeichnet, dass ein Insulin, vorzugsweise Humaninsulin oder Schweineinsulin abgetrennt wird.

6. Verfahren gemäss der Ansprüche 1 bis 4, dadurch gekennzeichnet, dass ein Insulinderivat, insbesondere ein Insulinester abgetrennt wird.

7. Verfahren gemäss Anspruch 6, dadurch gekennzeichnet, dass Humaninsulin-B30-di-tert-butyl-threonin abgetrennt wird.

8. Verfahren gemäss Anspruch 7, dadurch gekennzeichnet, dass die Abtrennung aus einem Gemisch erfolgt, das Schweineinsulin und Trypsin enthält, und insbesondere einem solchen, wie es bei der enzymatischen Umwandlung von Schweineinsulin in Humaninsulin vorkommt.

9. An einem nicht mit organischen Resten derivatisierten Kieselgel mit einer Korngrösse $\geq$ 40 $\mu$m säulenchromatographiertes Insulin, insbesondere Humaninsulin oder Schweineinsulin.

10. An einem nicht mit organischen Resten derivatisierten Kieselgel mit einer Korngrösse $\geq$ 40 $\mu$m säulenchromatographierter Insulinester, insbesonndere Humaninsulin-B30-di-tert-butyl-threonin.

## Claims

1. A process for the chromatographic separation of mixtures of insulin, insulin derivatives and, where appropriate, contaminations, characterized in that the mixtures are cromatographed on a column filled with silica gel, the silica gel not having been subjected to derivatisation by organic radicals and having a grain size of $\geq$ 40 $\mu$m.

2. A process according to claim 1, characterized in that the silica gel used has a grain size of 63–125 $\mu$m.

3. A process according to claim 1 or 2, characterized in that the silica gel used has an average pore diameter of 60 Å.

4. A process according to anyone of claims 1 to 3, characterized in that it is chromatographed with an eluent consisting of chloroform, methanol, water, triethylamin and, if desired, formic acid, the eluent advantageously consisting of 1500–2100 parts by volume of chloroform, 1000–1500 parts by volume of methanol, 350–450 parts by volume of water, 35–55 parts by volume of triethylamin and 0–15 parts by volume of formic acid.

5. A process according to anyone of claims 1 to 4, characterized in that an insulin, preferably human insulin or porcine insulin is separated.

6. A process according to anyone of claims 1 to 4, characterized in that an insulin derivative, in particular an insulin ester is separated.

7. A process according to claim 6, characterized in that human insulin B$^{30}$-di-tert.-butyl-threonine is separated.

8. A process according to claim 7, characterized in that the separation is effected from a mixture which contains porcine insulin and trypsin, and in particular from a mixture as is obtained with the enzymatic conversion of porcine insulin into human insulin.

9. An insulin chromatographed on a column of a silica gel having a grin size of $\geq$ 40 $\mu$m and not having been subjected to a derivatisation with organic radicals, in particular human insulin or porcine insulin.

10. An insulin ester chromatographed on a column of a silica gel having a grain size of $\geq$ 40 μm and not having been subjected to a derivatisation with organic radicals, in particular human insulin-B$^{30}$-di-tert.-butyl-threonine.

## Revendications

1. Procédé pour la séparation chromatographique de mélanges d'insuline, de dérivés d'insuline et d'impuretés éventuelles, caractérisé en ce que l'on chromatographie les mélanges sur une colonne garnie de gel de silice, le gel de silice n'étant pas sous forme de dérivé avec des restes organiques et ayant une dimension granulométrique supérieure à 40 μm.

2. Procédé selon la revendication 1, caractérisé en ce que le gel de silice utilisé a une dimension granulométrique de 63 à 125 μm.

3. Procédé selon l'une des revendications 1 et 2, caractérisé en ce que le gel de silice utilisé à un diamètre de pores moyen de 60 Å.

4. Procédé selon l'une quelconque des revendications 1 à 3, caractérisé en ce que l'on effectue la chromatographie avec un mélange solvant constitué de chloroforme, de méthanol, d'eau, de triéthylamine et éventuellement d'acide formique, le mélange solvant contenant de préférence de 1500 à 2100 parties en volume de chloroforme, de 1000 à 1500 parties en volume de méthanol, de 350 à 450 parties en volume d'eau, de 35 à 55 parties en volume de triéthylamine et de 0 à 15 parties en volume d'acide formique.

5. Procédé selon l'une quelconque des revendications 1 à 4, caractérisé en ce que l'on sépare une insuline, de préférence l'insuline humaine ou l'insuline de porc.

6. Procédé selon l'une quelconque des revendications 1 à 4, caractérisé en ce que l'on sépare un dérivé d'insuline, de préférence un ester d'insuline.

7. Procédé selon la revendication 6, caractérisé en ce que l'on sépare l'ester de B30-di-tert-butyl-thréonine de l'insuline humaine.

8. Procédé selon la revendication 7, caractérisé en ce que l'on effectue la séparation à partir d'un mélange qui contient de l'insuline de porc et de la trypsine, et en particulier d'un mélange tel qu'il se présente lors de la conversion enzymatique de l'insuline de porc en insuline humaine.

9. Insuline, en particulier insuline humaine ou insuline de porc, chromatographiée sur une colonne de gel de silice ne formant pas de dérivés avec des radicaux organiques et ayant une dimension granulométrique supérieure à 40 μm.

10. Ester d'insuline, en particulier ester de B30-di-tert-butyl-thréonine de l'insuline humaine chromatographié sur une colonne de gel de silice n'étant pas sous forme de dérivé avec des restes organiques et ayant une dimension granulométrique supérieure à 40 μm.